# EUROPEAN PATENT SPECIFICATION

(11) **EP 3 725 256 B1**
(45) Date of publication and mention of the grant of the patent: **30.08.2023**
(21) Application number: 19169439.7
(22) Date of filing: 16.04.2019
(51) Int. Cl.: A61B 90/00, A61B 34/20

(54) **TRACKER FOR SURGICAL NAVIGATION**
VERFOLGER FÜR CHIRURGISCHE NAVIGATION
SUIVEUR DE NAVIGATION CHIRURGICALE

(43) Date of publication of application: 21.10.2020
(73) Proprietor: Stryker European Operations Limited, Carrigtwohill, Co. Cork T45 HX08 (IE)
(72) Inventor: GAMM, Gerd Ulrich, 79104 Freiburg (DE); UMBDENSTOCK, Emeric, 79106 Freiburg (DE); HERRMANN, Florian, 77963 Schwanau (DE)
(74) Representative: Röthinger, Rainer

(56) References cited:
- EP-A1- 2 944 283
- WO-A1-2014/116961
- WO-A1-2019/018702
- CN-A- 106 207 540
- JP-A- H05 180 917
- KR-A- 20170 138 806
- US-A1- 2004 147 839
- US-A1- 2016 165 719

## Description

### Technical Field

The present disclosure generally relates to a tracker for surgical navigation. In particular, a tracker comprising a deformable patch is presented.

### Background

Many surgical procedures benefit from determining a position and orientation (i.e., a pose) of surgical objects, such as a surgical instrument and a patient, in an operating room. A surgical navigation system can be employed to track the surgical instrument relative to the patient and to calculate a current pose of the surgical instrument relative to registered three dimensional image data of the patient.

In a typical application of a surgical navigation system, the surgical instrument and the patient are each associated with a tracker, wherein three dimensional image data previously obtained by, for example, a computer tomography (CT) scan are initially registered with a pose of the patient tracker. By then continuously tracking the patient and the surgical instrument, the surgical navigation system can determine the pose of both surgical objects and calculate a spatial relationship between the surgical instrument and the three dimensional image data. The determined spatial relationship can, for example, be displayed on a screen, helping the surgeon guide the surgical instrument relative to the patient.

Trackers that are adapted to be attached to a skin surface of the patient often comprise a flexible patch carrying one or more light sources. It has been found that the shape of the skin surface differs from patient to patient, which may require a certain deformation of the patch upon applying same to the patient. Furthermore, the skin surface may be deformed during the surgery, e.g., due to movement of body parts or when opening an incision at the site of surgery. A deformation of the skin surface may, however, lead to a partial detachment of the tracker patch attached thereto, so that surgical navigation will be impaired. A deformation of the tracker patch may additionally impose a strain on electrical wiring of the light sources supported by the patch, especially when such wiring is also fixedly attached to the patch. Moreover, such wiring may prevent deformation of the patch or break when being strained.

The document EP 2944283 A1 discloses a navigation system for tracking the position of a work target. The navigation system can detect distortions of a trackable device with a flexible substrate during the navigation procedure and compensate for such deformation in a way that reduces or eliminates navigational error and/or avoids or reduces the need to re-set the navigation system during a navigation procedure.

The document US 2004/147839 A1 discloses a method of registering an article having a surface to previously created scan data of the article. The method includes the steps of providing a flexible substrate having multiple tracking points attached to the substrate, applying the substrate to the article to be registered, creating a model of the surface of the article from a location of each tracking point, and registering the model with the previously created scan data.

The document US 2016/0165719 A1 discloses an electronic patch comprising a foldable circuit layer that includes a foldable network comprising: a plurality of electronic modules comprising a plurality of electronic components, and flexible straps that connect the plurality of electronic modules, wherein the flexible straps comprise conductive circuits that are conductively connected with the plurality of electronic components in the plurality of electronic modules. Neighbouring electronic modules can undulate in opposite directions normal to the foldable circuit layer.

The document WO 2014/116961 A1 discloses a surgical instrument having an elongated body portion having a proximal end and a distal end. The body portion is formed from a plastically deformable material such that the body portion can be bent between the proximal and distal ends from a first configuration to a second bent configuration and maintains the bent configuration. A flexible circuit sheet having at least a pair of lead wires disposed around the body portion. The pair of lead wires are configured to conform to the bent configuration of the body portion such that they do not break during bending of the body portion.

### Summary

There is a need for a tracker that solves one or more of the aforementioned or other problems.

According to one aspect, a tracker for surgical navigation is provided. The tracker comprises a patch comprising multiple fingers and being at least one of stretchable and compressible in at least one direction, and a plurality of tracker elements selected from a light source or a coil, the tracker elements being supported by the patch. The tracker further comprises a plurality of electrical connections electrically coupled to the tracker element and supported by the patch, wherein at least two of the fingers each support at least one tracker element and at least one electrical connection thereof, the electrical connections having a meandering configuration along the direction in which the patch is at least one of stretchable and compressible.

The patch may be planar. The patch may be deformable in only one direction (i.e., uni-directionally). The patch may be deformable in any direction of an imaginary plane comprising the patch. The patch may be deformable isotropically. The patch may be bendable.

The patch may be elastically deformable. The patch is stretchable and/or compressible. The patch may have a thickness between 0,05 mm and 2 mm. For example, the patch may have a thickness between 0,2 and 1 mm.

The meandering shape may be defined by the electrical connection repeatedly (e.g., more than three, five, ten or twenty times) crossing along its extension an imaginary line. That line may be straight or curved, as long as the radius of curvature of the line is smaller than a characteristic feature (e.g., a curvature) defining the meandering shape.

The meandering shape may comprise a periodic or aperiodic pattern. The periodic pattern may extend over a part or over the entire extension of the electrical connection. The periodic pattern may lie in a plane spanned by a planar patch.

The meandering shape may comprise at least one of a wave pattern, a rectangular wave pattern and a zigzag pattern. The meandering shape may have a single pattern that repeats itself. The meandering shape may have a plurality of different patterns. The plurality of different patterns may be arranged in series and/or may be superimposed.

The electrical connections may have a width between 5µm and 50 µm. For example, the electrical connections may have a width between 10 µm and 40 µm, preferable between 15 µm and 30 µm.

The tracker comprises a plurality of tracker elements and a plurality of electrical connections. The plurality of tracker elements may comprise one or more light sources and/or one or more coils. At least some of the tracker elements and electrical connections may be alternatingly electrically coupled in series. At least some of the electrical connections may be connected in parallel to at least some of the tracker elements. The tracker may comprise a junction of a plurality of electrical connections. The tracker may comprise a controller electrically connected with at least two of the tracker elements and configured to control operation of the at least two tracker elements independently. Independent operation of the at least two tracker elements may comprise at least one of providing different operation currents (including no current), different voltages and different operation frequencies.

The patch may have a meandering shape. The patch comprises multiple fingers. At least two of the fingers each support at least one tracker element and at least one electrical connection thereof. Each finger may support at least one tracker element and at least one electrical connection thereof.

At least a part of the patch may have the shape of a frame enclosing a central opening. The frame shape may be rectangular, elliptical, circular or polygonal.

The electrical connection may be printed onto the patch. Alternatively, or additionally, the electrical connection may comprise a wire.

The tracker may comprise a plurality of layers, wherein the electrical connections are arranged in at least one of the plurality of layers. The plurality of layers may be arranged at at least one of a top surface of the patch, a bottom surface of the patch and embedded inside the patch. At least some of the electrical connections may be arranged in different layers and/or at least some of the electrical connections may be arranged in a same layer. The tracker may comprise a via (also called vertical interconnection access) configured to electrically connect at least two of the plurality of layers. The electrical connection may be electrically connected with the via. The via may be electrically connected to multiple electrical connections that are arranged in separate layers.

The tracker may comprise an adhesive configured to attach the patch to a surface of a surgical object. The adhesive may be applied to the patch by printing or otherwise.

According to a second aspect, a method for operating a tracker for surgical navigation is provided. The tracker comprises a patch being deformable in at least one direction, at least two tracker elements selected from one or more light sources and one or more coils supported by the patch, and an electrical connection electrically coupled to each tracker element and supported by the patch, at least one of the electrical connections having a meandering configuration along the direction in which the patch is deformable. The method comprises controlling operation of the at least two tracker elements independently.

### Brief Description of the Drawings

Further details, advantages and aspects of the present disclosure will become apparent from the following embodiments taken in conjunction with the drawings, wherein:
- Fig. 1A: shows a first embodiment of a tracker in a non-deformed configuration;
- Fig. 1B: shows the tracker of Fig. 1A in a deformed configuration;
- Fig. 1C: shows a second embodiment of a tracker in a non-deformed configuration;
- Fig. 1D: shows the tracker of Fig. 1C in a deformed configuration;
- Figs. 2A-2F: show different examples of meandering shapes for the electrical connection of the tracker;
- Fig. 3: shows a cross section of a third embodiment of a tracker;
- Figs. 4A-C: show different examples of circuit diagrams for electrically connecting a plurality of light sources of a tracker with electrical connections;
- Fig. 5: show a top view of a fourth embodiment of a tracker;
- Figs. 6A, B: show a top view of a fifth embodiment of the tracker in a non-deformed and a deformed configuration, respectively;
- Fig. 7: a top view of a sixth embodiment of a tracker; and
- Fig. 8: a flow diagram of a method for operating a tracker for surgical navigation.

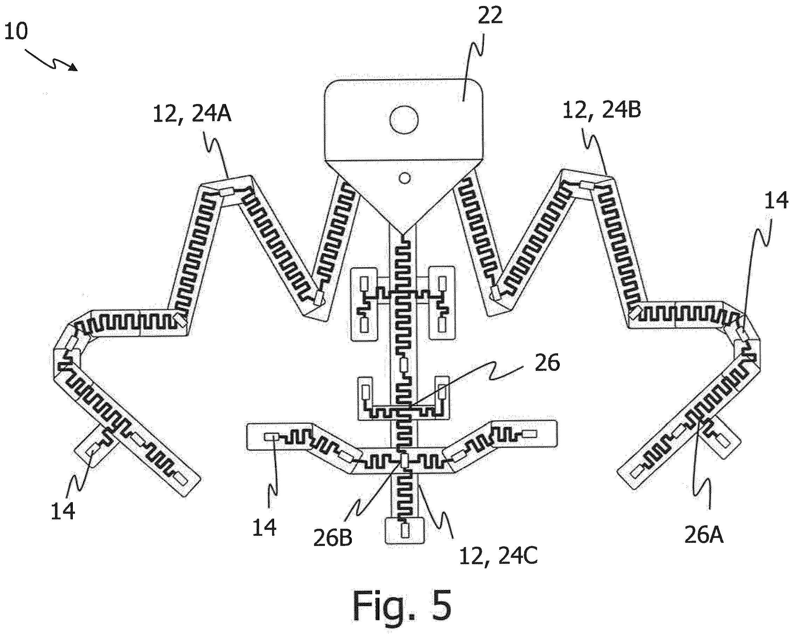

An embodiment of the invention is depicted in figure 5.

### Detailed Description

In the following description, exemplary embodiments of a tracker for surgical navigation and a method for operating a tracker will be explained with reference to the drawings. The same reference numerals will be used to denote the same or similar structural features.

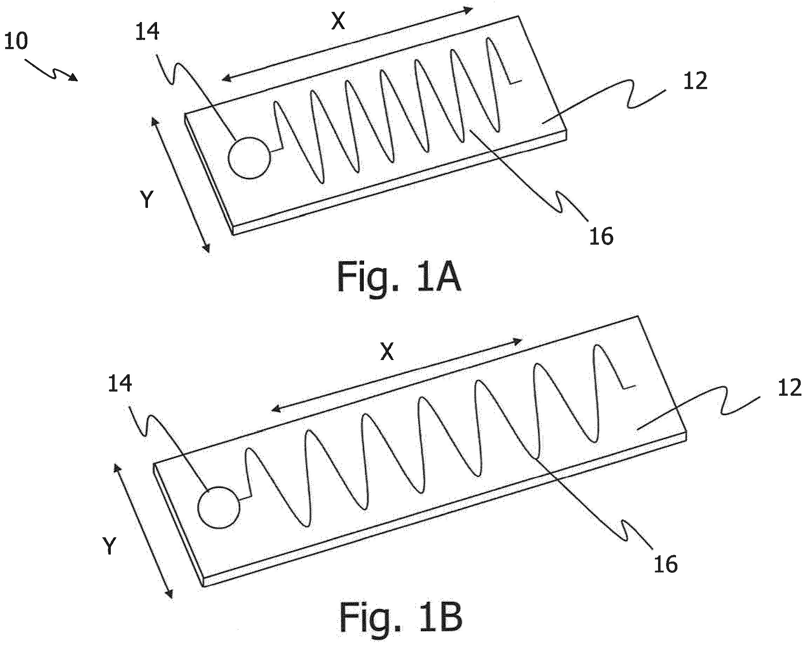

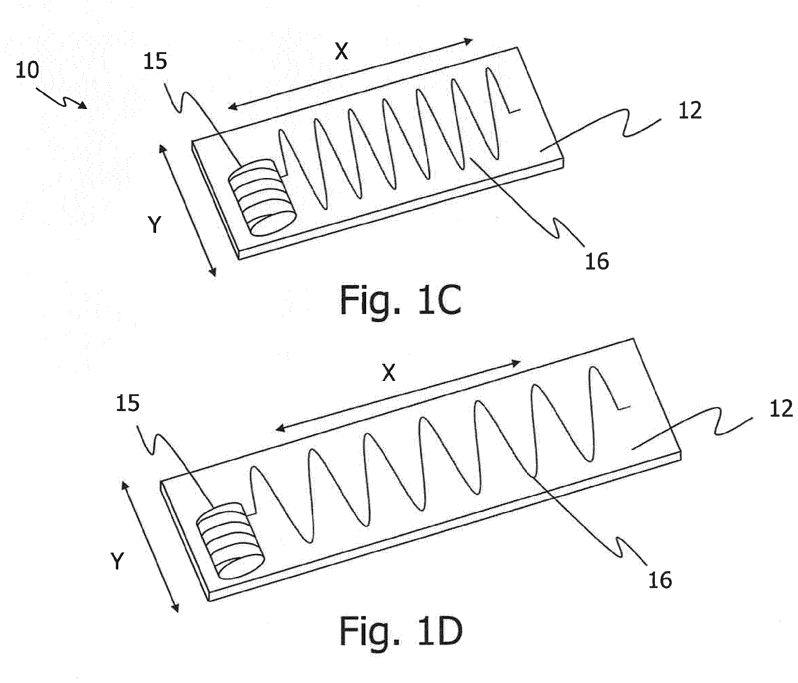

Fig. 1 shows a first embodiment of a tracker 10 in a non-deformed configuration. The tracker 10 comprises a patch 12. The patch 12 comprises a deformable material. The patch 12 may, for example, comprise at least one of polyimide (PI), liquid crystal polymer (LCP) and polyurethane (PU), such as thermoplastic PU (TPU). Additionally or alternatively, the patch 12 may comprise other materials that result in a deformability of the patch 12, such as rubber or textile. The patch 12 may comprise an open-pored and/or closed-pored foamed material. The patch 12 may be manufactured as a molded article.

The patch 12 is elastically deformable and therefore (at least partially) configured to return to its original shape after a deforming force is no longer applied. In particular, the patch 12 is stretchable and/or compressible. The patch 12 may be stretchable by more than 10%, more than 20% or more than 30% before it breaks.

The patch 12 shown in Fig. 1A is stretchable in a direction X marked by a double-arrow. Additionally, the patch 12 may be stretchable in a plurality of further directions (e.g., in the direction X and in a direction Y perpendicular to direction X). The patch 12 may be stretchable in any direction of an imaginary plane (e.g., the plane defined by the two orthogonal directions X and Y). The patch 12 may further be stretchable isotropically (i.e., uniformly in all orientations). The deformability of the patch 12 allows the patch 12 to adapt to forces applied to the patch 12 when attached to a surgical object, such as a surface of a patient (not shown). Such forces may occur due to active and/or passive movement of the patient body or during movement of the skin of the patient (e.g., upon opening an incision close to the patch 12).

The patch 12 shown in Fig. 1A has a flat and planar shape. Therefore, obstruction of the tracker 12 caused at the surgery site is reduced. Alternatively, the patch 12 may have a non-planar shape that is configured to adapt to geometrical features of the surgical object (e.g., anatomical patient features such as nose or chin).

The tracker 10 further comprises tracker element in form of a light source 14 supported by the patch 12. The light source 14 may comprise at least one of a light emitting diode (LED), an organic light emitting diode (OLED), a laser, an incandescent light source and an optical fibre. The tracker 10 may comprise a plurality of light sources 14. With a larger number (such as two, three, four, or more) of light sources 14, the amount of trackable degrees of freedom and/or the tracking accuracy can be increased. The light source 14 is supported by the patch 12. The light source 14 shown in Fig. 1A is arranged on top of the patch 12. Alternatively, the light source 14 may be arranged in an opening in the patch or embedded inside the patch 12, wherein the patch 12 is at least partially translucent for light emitted by the light source 14. The light source 14 is configured to emit light, which can be detected by an optical surgical navigation system as known in the art. Based on the detected light, the tracker 10 can be tracked by the surgical navigation system. The tracker 10 is therefore a surgical navigation tracker configured to be used for surgical navigation.

A surgical navigation system (not shown) comprises the tracker 10 and a sensor system (not shown) configured to detect information indicative of at least one degree of freedom (e.g., position and orientation) of the tracker 10. To this end, the sensor system may comprise a camera configured to detect light emitted by a light source 14 of the tracker 10.

The tracker 10 further comprises an electrical connection 16 electrically coupled to the light source 14. The electrical connection 16 may comprise or consist of copper, nickel, silver or gold, or an alloy thereof. The electrical connection 16 shown in Fig. 1A is printed onto the patch 12. Alternatively, the electrical connection 16 comprises a wire that is attached to and/or embedded into the patch 12. When the patch 12 is deformed (e.g., stretched along direction X), the electrical connection 16 stretches along with the patch 12.

The electrical connection 16 illustrated in Fig. 1A has a meandering configuration along the direction in which the patch 12 is deformable (e.g., direction X in Fig. 1A). The meandering shape defined by the electrical connection repeatedly (i.e., more than ten times) crosses along its extension an imaginary straight line that extends parallel to the direction X and defines a longitudinal axis of the patch 12. It will be appreciated that in other embodiments this line may be curved, as long as its radius of curvature is significantly larger than a characteristic size of a geometric pattern underlying the meandering configuration.

Fig. 1B shows the tracker 10 of Fig. 1A in a deformed configuration, wherein the patch 12 is stretched along direction X. If the electrical connection 16 only extended straight, the electrical connection 16 would not be able to stretch during the deformation of the patch 12 and possibly break and/or hinder the deformation of the patch 12. Instead, the electrical connection 12 has a meandering shape that unfolds when the patch 12 is stretched. While unfolding, portions of the electrical connection 16 that do not extend in the deformation direction X partially rotate about an imaginary point in the center of the meandering shape, which essentially lengthens the electrical connection in direction X.

Similarly, in the case the patch 12 is compressed (not shown), the electrical connection 16 further folds onto itself and compresses along with the patch 12. The meandering shape of the electrical connection essentially provides an elastic wiring for the tracker 10.

Fig. 1C shows a second embodiment of a tracker 10 in a non-deformed configuration. The second embodiment differs from the first embodiment essentially in the tracker element comprising a coil 15 instead of a light source 14. The coil 15 is supported by the patch 12 and electrically coupled to the electrical connection 16.

The coil 15 allows tracking of the tracker 10 via an electromagnetic tracking principle. One way to apply the electromagnetic tracking principle is to provide an electromagnetic field generator (not shown) that generates an electromagnetic field that induces a current in the coil 15. The current or a signal indicative of the induced current may be measured by a measuring device (not shown). Based on the current or signal, at least a part of a position and orientation of the coil 15 may be determined. By providing a plurality of coils 15, more degrees of freedom may be determined. Usually, a tracker 10 comprising two coils 15 arranged skewed relative to each other allows determining the position and orientation of the tracker 10.

A surgical navigation system (not shown) may be provided that comprises the tracker 10 and a sensor system (not shown) configured to detect information indicative of at least one degree of freedom (e.g., position and orientation) of the tracker 10. The sensor system may comprise a measuring device configured to detect a current and/or voltage induced in the coil 15 of the tracker 10. To this end, the surgical navigation system may comprise a field generator configured to generate an electric field that is capable of inducing a current in the coil 15 of the tracker 10.

The tracker 10 may comprise a plurality of coils 15. Furthermore, the tracker 10 may comprise at least one coil 15 and at least one light source 14. A tracker 10 with light sources 14 and coils 15 may be used as a hybrid-tracker that can be tracked optically as well as electromagnetically. The sensor system of the surgical navigation system may comprise the measuring device as described above and a camera configured to detect light emitted by a light source 14 of the tracker 10. Such a surgical navigation system is capable of tracking electromagnetically and optically.

The meandering shape of the electrical connection shown in Figs. 1A and 1B essentially describes a periodic wave form similar to a sine wave. Alternatively, the meandering shape may comprise an aperiodic pattern.

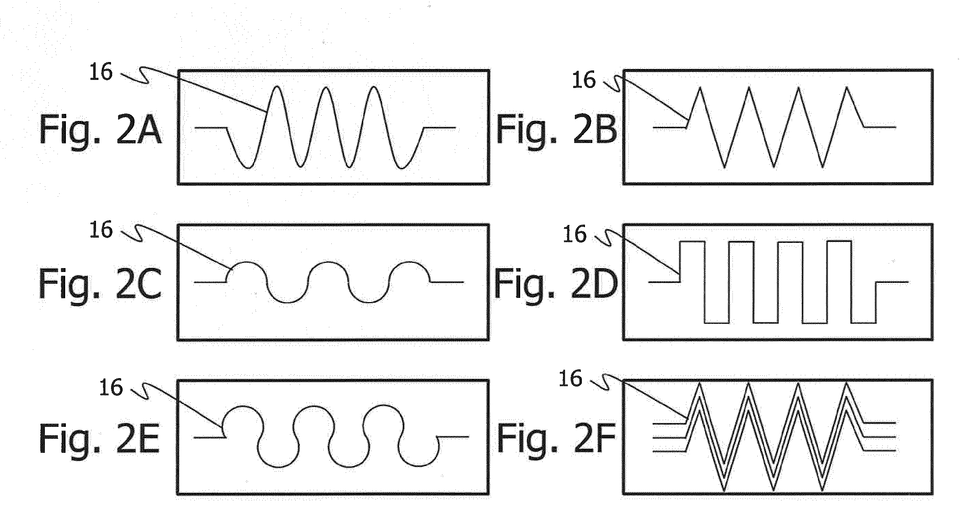

Figs. 2A to 2F show further examples of periodic wave patter according to embody-ments of the present disclosure. Fig. 2A shows an electrical connection 16 with a wave pattern (e.g., a sinus wave pattern). Such a wave pattern has no acute angles and therefore provides reduction of local force peaks during deformation, which increases the lifetime of the electrical connection 16.

Fig. 2B shows a zigzag pattern. Such a pattern comprises straight portions which may be easier to print and/or manufacture.

Fig. 2C shows a wave pattern comprising circular arcs. The constant curvature of the arcs may be simple to manufacture and provide an even force distribution.

Fig. 2D shows a rectangular wave pattern. The rectangular wave pattern provides a larger degree of folding. In present disclosure, the degree of folding is understood as a ratio of the entire length between two end points of a straightened electrical connection 16 (also called arc length) and a distance between the two end points of the electrical connection 16 in an unbiased meandering configuration. A wave pattern with a large degree of folding comprises a larger amount of portions that can be straightened during stretching. On the other hand, a straight line has a smallest degree of folding with a ratio of one. The rectangular wave pattern has a large degree of folding (compared to the wave pattern shown in Fig. 2C), and can therefore be stretched further apart.

Fig. 2E shows a wave pattern comprising circular arcs that extend over more than 180°. The pattern provides a larger degree of folding and an even force distribution.

Some wave patterns (in particular wave patterns with a low degree of folding) are stackable next to each other in such a way that a plurality of electrical connections 16 with such a pattern can extend parallel to each other. Fig. 2F shows an example of such a parallel extension with a zigzag pattern as shown in Fig. 2B. A similar configuration is also possible with other wave pattern, such as the wave pattern shown in Figs. 2A and 2C. Generally, such a parallel extension can be manufactured with wave forms that do contain no (or few) portions that extend perpendicular or backwards relative to a main extension of the electrical connection 16.

The examples of wave forms shown in Figs. 2A to 2F are periodic and contain a single wave form. Alternatively, the meandering shape of the electrical connection may comprise a plurality of different pattern, or wave forms. The different wave forms may be arranged in series and/or superimposed (not shown). Furthermore, the meandering shape may not be periodic. The degree of folding may vary along the extension of the electrical connection 16, which allows customizing the deformability of the electrical connection 16.

The tracker 10 may comprise a plurality of electrical connections 16 that need to be arranged close to each other. Such an arrangement may for example be required when providing a supply line and a return line for a light source 14, or when electrical connections 16 for a plurality of light sources 14 are necessary. Due to its meandering shape, the electrical line 16 extends into a width direction (e.g., Y direction in Figs. 1A and 1B), which needs to be taken into account when arranging a plurality of electrical connections 16 close to each other. One possibility to arrange a plurality of electrical connections is to stack similarly shaped meandering electrical connections 16 next to each other as, for example, shown in Fig. 2F.

Another (additional or alternative) approach is to provide a plurality of layers in the tracker 10, in which electrical connections 16 are arranged.

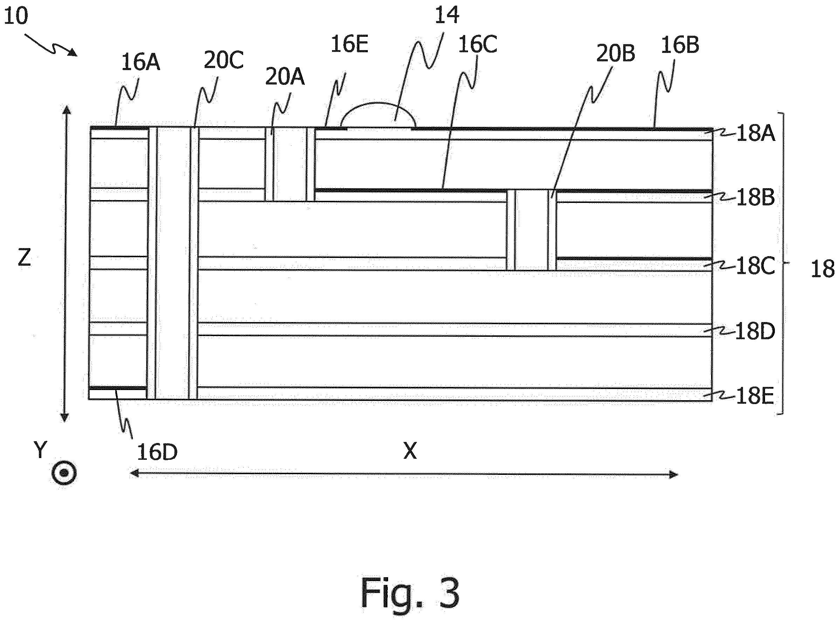

Fig. 3 shows a cross section of a third embodiment of a tracker 10. The tracker 10 may have any of the configurations presented, for example, in Figs. 1A to 1D and 2A to 2F above and also in the Figures that follow.

The tracker 10 comprises a patch 12 deformable in one or more directions of an imaginary plane defined by two orthogonal X and Y directions. Perpendicular to the X and Y directions extends a Z direction. Along the Z direction, the tracker 10 comprises a plurality of layers 18. The tracker 10 shown in Fig. 3 comprises five layers 18A-18E, wherein some layers comprise at least one electrical connection 16. Alternatively, the tracker 10 may comprise any other number of layers and each layer 18A-18E may comprise any number of electrical connections 16 (including the case of no electrical connection 16 in a layer 18).

The tracker 10 shown in Fig. 3 comprises a light source 14 that is arranged on top of a first layer 18A. The tracker 10 comprises a plurality of vias 20 (also called vertical interconnection access). Each via 20 forms an electrical connection between two or more layers 18 of the tracker 10. The vias 20 may extend through the entire patch 12, such as via 20C which electrically connects all five layers 18. Alternatively, a via 20 may extend only to an inner layer 18 (also called blind vias), such as via 20A electrically connecting layers 18A and 18B. Further alternatively, a via 20 may extend from an inner layer 16 to another inner layer 16, such as via 20B (also called buried via), which extends from layer 18B to layer 18C.

The vias shown in Fig. 3 have through holes that extend through the each respective via 20. Alternatively, the vias 20 may be solid.

The electrical connections 16 from different layers are electrically connected by the vias 20. For example, via 20C is configured to electrically connect an electrical connection 16A arranged in the top layer 18A with an electrical connection 16D arranged in the bottom layer 18E.

Similarly, via 20A electrically connects an electrical connection 16E arranged in the top layer 18A with an electrical connection 16C arranged in layer 18B. In this way, the first light source 14A is provided with a supply line comprising the electrical connection 16B and a return line that comprises the electrical connections 16E, 16C and the via 20A.

The use of the vias 20 is not limited to electrically connecting electrical connections 16. Vias 20 may also electrically connect other electronic components of the tracker 10, such as a light source 14 or a power supply (not shown).

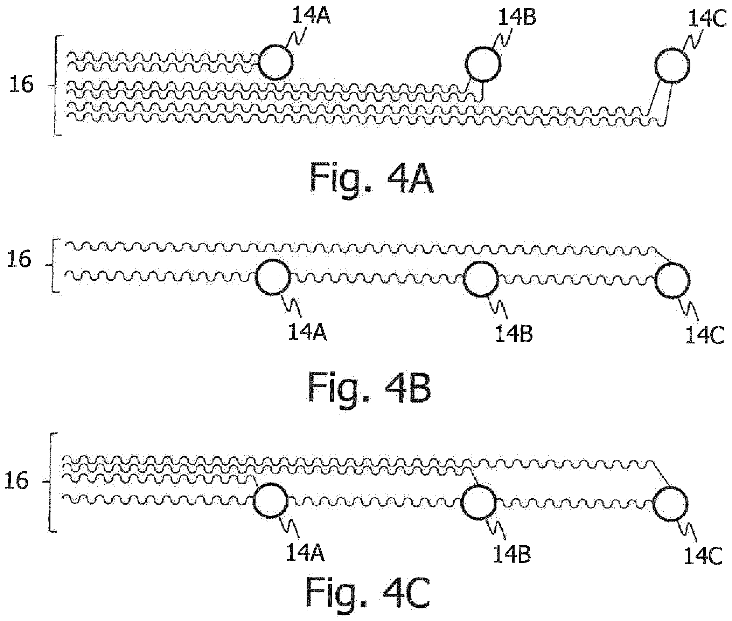

Figs. 4A-C show different examples of circuit diagrams for electrically connecting a plurality of light sources 14 of a tracker 10 with electrical connections 16. The electrical connections 16 shown in Figs. 4A-C comprise a periodic wave pattern similar to the one shown in Fig. 2C. However, the circuit examples shown in Figs. 4A-C are not limited thereto and every other meandering shape in any combination can be used for such circuits. Furthermore, the electrical connections 16 shown in Figs. 4A-C are arranged in the same layer. However, a plurality of layers may be provided, wherein any electrical connection 16 can be arranged in any layer of the plurality of layers. Electrical connections between layers can be realized with vias (e.g., the vias shown in Fig. 3).

Fig. 4A shows a circuit diagram, in which for every light source 14A-C a separate supply and return line (comprising electrical connections 16) is provided. In case of failure of one of the electrical connections 16 (e.g., breaking of an electrical connection 16) for one of the light sources 14, or in case of failure of one of the light sources 14, the supply and return lines of the remaining light sources 14 remain unaffected and continue to provide operability of the remaining light sources 14.

Fig. 4B shows a circuit diagram, in which the light sources 14A-C and electrical connections 16 are alternatingly electrically coupled in series. Such a circuit has low complexity and reduces the amount of required electrical connections 16.

Fig. 4C shows a circuit diagram comprising parallel and series circuits. The light sources 14A-C are connected in series, wherein for each light source 14A, B a separate return line coupled in parallel is provided. Such parallel circuits reduce the currents required for operation of a tracker 10 and permit an individual operation (e.g., on/off) of each light source 14A-C.

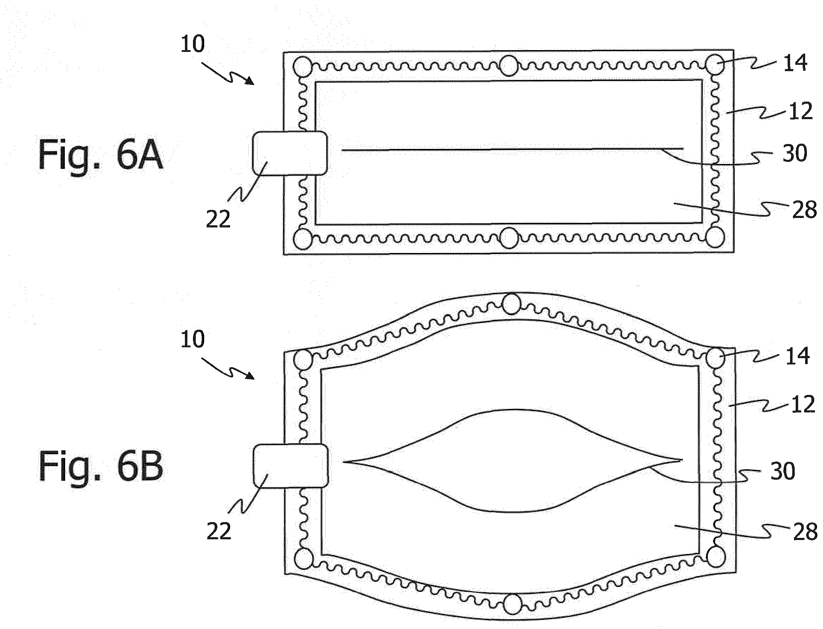

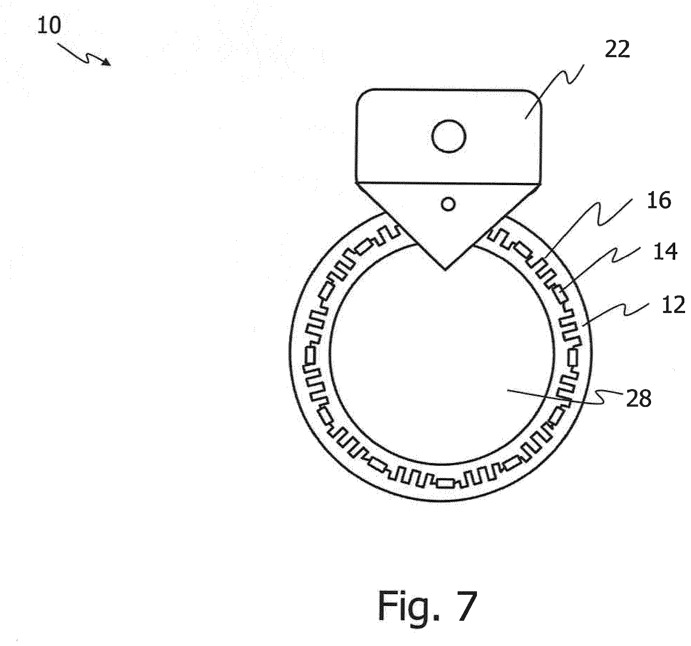

Figs. 5 to 7 show top views of different embodiments of a tracker 10 with series connections comprising the light sources 14 and the electrical connections 16. This series connection partly owes to the fact that such circuits are easier to illustrate in a drawing. Any other circuit configuration comprising series and/or parallel circuits (e.g., as shown in Figs. 4A-C) may be used instead. Furthermore, the embodiments shown in Figs. 5 to 7 have electrical connections 16 with a wave pattern (Figs. 6A, 6B) and a rectangular wave pattern (Figs. 5 and 7). However, these embodiments are not limited thereto. Alternatively, any other meandering shape (e.g., the patterns shown in Figs. 2A-2F) in any combination can be used.

Fig. 5 shows a top view of a fourth embodiment of a tracker 10. The tracker 10 comprises a deformable patch 12, a plurality of light sources 14, and a plurality of electrical connections 16 coupled to the light sources 14 and supported by the patch 12. The electrical connections 16 each have a meandering configuration.

The tracker 10 comprises a controller 22. The controller 22 is electrically connected with the electrical connections 16 (and consequently also with the light sources 14). The controller 22 comprises a power supply (not shown) that is configured to provide electrical power to the light sources 14. The power supply may comprise at least one of a rechargeable or non-rechargeable battery, a capacitor, a receiver for wireless energy transfer, and a connector to a power outlet. The controller 22 is configured to operate the light sources 14. Light source operation comprises selectively providing electrical power (e.g., providing power or not providing power) to a light source 14. The operation may further comprise controlling at least one of an operation frequency, operation current, and operation voltage. The controller 22 may, for example, be configured to provide an operation current between 1 to 10 mA. Particularly for providing such low currents, the electrical connections 16 are not required to have a large width. The electrical connections may have a width between 10µm to 50µm. Electrical connections 16 with such a low width can be easily deformed and are particularly suited for a meandering shape that can unfold.

The controller 22 is configured to operate at least two light sources 14 independently. For example, the controller 22 is configured to provide an operation current to only one light source 14 and not to another light source 14. The independent operation may further comprise at least one of a different operation frequency, different operation current, and different operation voltage. Independent control of different light sources 14 allows applying different functionality to different light sources 14 (e.g., one light source is used for indicating that the controller 22 is operational and another light source 14 is used for tracking), selecting different trackable light patterns, or tracking with two separate optical navigation system (e.g., operating at different operation frequencies or operating different types of light sources with different wave lengths).

The patch comprises multiple fingers 24A-C, wherein each of the fingers 24A-C branches out into sub fingers. The fingers 24A and C have a meandering shape. Each of the fingers 24A-C (and its corresponding sub fingers) supports at least one light source 14 and electrical connections 16 coupled with the light sources 14. In order to supply electrical power to the light sources 14 supported by the sub fingers, the tracker 10 comprises junctions 26 of a plurality (i.e., at least two) of the meandering electrical connections 16. Such a junction 26 may be located at a position of an electrical connection 16 at which another electrical connection 16 branches out (e.g., junction 26A). A junction 26 may also be located at a light source 14 (e.g., junction 26B). Furthermore, the junction may comprise a via (not shown).

When being attached to an uneven surface, such as a face of the patient, a tracker 10 comprising fingers 24A-C can adapt well to the shape of the surface.

Figs. 6A, B show a top view of a fifth embodiment of a tracker 10 in a nonbiased (Fig. 6A) and a deformed (Fig. 6B) configuration. The tracker 10 comprises a plurality of light sources 14 supported by a deformable patch 12. The patch 12 has the shape of a rectangular frame enclosing a central opening 28. The tracker 10 shown in Figs. 6A, B is attached to a skin surface of a patient (e.g., by an adhesive), wherein the opening 28 is arranged over an incision 30 made in the surface of the patient. The incision may, for example, be an abdominal incision.

The patch 12 comprises a plurality of electrical connections 16 that are coupled in series with the plurality of light sources 14. The electrical connections 16 could also be configured otherwise (see, e.g., Figs. 3 and 4 A-C). The tracker 10 further comprises a controller 22 electrically connected with the light sources 14 and configured to operate the light sources 14.

When opening the incision in order to access the surgery site, skin around the incision deforms and is mainly pushed in a direction away from the incision. Since the tracker 10 is attached to the skin of the patient, a force is acting on the tracker 10. The patch 12 and the electrical connections 16 (due to their meandering shape) are deformable and can therefore adapt to the force by deformation. Fig. 6B shows the tracker 10 in a deformed configuration. In its deformed configuration, a distance between the light sources 14 is increased. The increased distance is compensated by a (partial) straightening of the meandering shapes of the electrical connections 16. From the deformed position illustrated in Fig. 6B, the tracker 10 may deform back to the non-deformed position illustrated in Fig. 6A when the incision is closed again, without impairing or interrupting the tracking process. Deviations of individual light sources 14 from their preceding position upon deformation or relaxation can easily be compensated using the technique presented in EP 2 944 283 A.

Fig. 7 shows a top view of a sixth embodiment of a tracker 10. The fifth and sixth embodiments both comprise a patch 12 with a shape of a frame enclosing a central opening 28. The fifth and sixth embodiments essentially differ in the shape of the frame. The tracker 10 has a patch 12 with a circular shape. Such a circular shape can be attached around circular body parts, such as a bent knee (not shown). One way of attaching the patch 12, is to pull the circular patch 12 over the bent knee. The patch 12 shown in Fig. 7 is elastic. Therefore, when the patch 12 is pulled over the knee, the patch 12 generates a bias inwards towards the knee, which allows the elastic patch 12 to be attached to the knee. The tracker 10 may comprise an adhesive for improving the attachment to the knee. The deformation of the patch 12 causes a distance between the light sources 14 to increase. As a result of their meandering shape, the electrical connections 16 have a deformability that allows the electrical connections 16 to adapt to the deformation of the patch 12 by stretching. The electrical connections 16 could and also be configured otherwise (see, e.g., Figs. 3 and 4A-C).

Figs. 3 to 7 show trackers 10 that comprise light sources 14. For the sake of conciseness, the drawings were not illustrated a second time with coils 15. It is noted that any of the trackers 10 shown in Figs. 3 to 7 may alternatively comprise at least one coil 15 instead of at least one of the light sources 14. Any tracker 10 described above and shown in the drawings may comprise exclusively light sources 14 or exclusively coils 15. Alternatively, the trackers 10 may comprise at least one light source 14 and at least one coil 15.

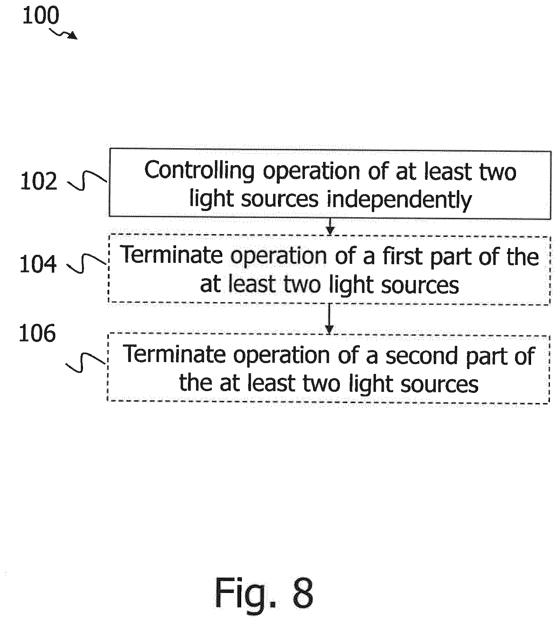

Fig. 8 shows a flow diagram of a method 100 for operating a tracker 10 for surgical navigation. The tracker 10 comprises a patch 12 being deformable in at least one direction, at least two tracker elements selected from one or more light sources 14 and one or more coils 15, the at least two tracker elements being supported by the patch 12, and at least one electrical connection 16 electrically coupled to each tracker element 14, 15 and supported by the patch 12. One or both of the electrical connections 16 have a meandering configuration along the direction in which the patch 12 is deformable. The tracker 10 may be any of the trackers 10 having a plurality of tracker elements 14, 15 described above.

The method 100 comprises a step 102 of controlling operation of the at least two tracker elements 14, 15 independently.

In case of controlling at least two light sources 14, controlling the operation independently comprises selectively providing a first electrical power to a first set of the at least two light sources 14 and a second electrical power to a second set of the at least two light sources 14, wherein the first and second electrical power can be different. Controlling the operation may further comprise adjusting for the at least two light sources 14 at least one of different operation frequencies, different operation currents, and different operation voltages. For example, an operation current between 1 to 10 mA may be provided to one or more of the light sources 14 that are to be tracked and a larger current may be provided to one or more of the light sources 14 that indicate that the tracker 10 is operating. Since detection means for optical navigation are becoming increasingly sensitive, the light sources 14 for tracking can emit with lower intensity and therefore require less operating current than light sources that need to be visible to the surgeon.

Controlling operation frequency independently for the at least two light sources 14 may comprise operating a first set of the at least two light sources 14 with a first frequency and operating a second set of the at least two light sources 14 with a second frequency, wherein the first and second operation frequency may be different. The first frequency may be synchronized with a first surgical navigation system and/or the second frequency may be synchronized with a second surgical navigation system.

The method may optionally comprise a step 104 of terminating operation of the first set of the at least two light sources 14. The method may optionally comprise a further step 106 of terminating operation of the second set of the at least two light sources 14.

The features described in relation to the exemplary embodiments shown in the drawings can be readily combined to result in different embodiments. It is apparent, therefore, that the present disclosure may be varied in many ways. Such variations are not to be regarded as a departure from the scope of the invention as defined by the claims appended hereto.

## Claims

1. A tracker (10) for surgical navigation, the tracker (10) comprising
a patch (12) being at least one of stretchable and compressible in at least one direction;
a plurality of tracker elements selected from a light source (14) or a coil (15), the tracker elements (14, 15) being supported by the patch (12); and
a plurality of electrical connections (16) electrically coupled to the tracker elements (14, 15) and supported by the patch (12), the electrical connections (16) having a meandering configuration along the direction in which the patch (12) is at least one of stretchable and compressible **characterised in that** the patch (12) comprises multiple fingers, wherein at least two of the fingers each support at least one tracker element and at least one electrical connection thereof.

2. The tracker (10) according to claim 1, wherein
the patch (12) is elastically deformable.

3. The tracker (10) according to claim 1 or 2, wherein
the meandering configuration comprises a periodic pattern.

4. The tracker (10) according to any preceding claim, wherein
the meandering configuration comprises at least one of a wave pattern, a rectangular wave pattern and a zigzag pattern.

5. The tracker (10) according to any preceding claim, wherein
the electrical connections (16) have a width between 5µm to 50 µm.

6. The tracker (10) according to any preceding claim, wherein
at least some of the tracker elements (14, 15) and electrical connections (16) are alternatingly electrically coupled in series.

7. The tracker (10) according to any preceding claims,
comprising a junction (26) of a plurality of electrical connections (16).

8. The tracker (10) according to any preceding claim,
comprising a controller (22) electrically connected with at least two of the tracker elements (14, 15) and configured to control operation of the at least two of the tracker elements (14, 15) independently.

9. The tracker (10) according to any preceding claim,
wherein the patch (12) has a meandering shape.

10. The tracker (10) according to any preceding claim,
wherein at least a part of the patch (12) has the shape of a frame enclosing a central opening (28); and/or
wherein the electrical connections (16) are printed on the patch (12).

11. The tracker (10) according to any preceding claim,
comprising a plurality of layers (18), wherein the electrical connections (16) are arranged in at least one of the plurality of layers (18);
wherein, as an option, the tracker comprises a via (20) configured to electrically connect at least two of the plurality of layers (18).

12. The tracker (10) according to any preceding claim,
comprising an adhesive configured to attach the patch (12) to a surface of a surgical object.

## Patentansprüche

1. Verfolger (10) für die chirurgische Navigation, wobei der Verfolger (10) umfasst
ein flächiges Element (12), das in zumindest einer Richtung dehnbar und/oder stauchbar ist;
eine Mehrzahl von Verfolgerelementen, ausgewählt aus einer Lichtquelle (14) oder einer Spule (15), wobei die Verfolgerelemente (14, 15) von dem flächigen Element (12) getragen werden; und
eine Mehrzahl von elektrischen Verbindungen (16), die elektrisch mit den Verfolgerelementen (14, 15) gekoppelt sind und von dem flächigen Element (12) getragen werden, wobei die elektrischen Verbindungen (16) eine mäandernde Konfiguration entlang der Richtung aufweisen, in der das flächige Element (12) dehnbar und/oder stauchbar ist, **dadurch gekennzeichnet, dass**
das flächige Element (12) mehrere Finger umfasst, wobei zumindest zwei der Finger jeweils zumindest ein Verfolgerelement und zumindest eine elektrische Verbindung hiervon tragen.

2. Verfolger (10) nach Anspruch 1, wobei
das flächige Element (12) elastisch verformbar ist.

3. Verfolger (10) nach Anspruch 1 oder 2, wobei
die mäandernde Konfiguration ein periodisches Muster umfasst.

4. Verfolger (10) nach einem der vorangehenden Ansprüche, wobei
die mäandernde Konfiguration zumindest eines von einem Wellenmuster, einem Rechteckwellenmuster und einem Zickzackmuster umfasst.

5. Verfolger (10) nach einem der vorangehenden Ansprüche, wobei
die elektrischen Verbindungen (16) eine Breite zwischen 5µm und 50 µm aufweisen.

6. Verfolger (10) nach einem der vorangehenden Ansprüche, wobei
zumindest einige der Verfolgerelemente (14, 15) und elektrischen Verbindungen (16) alternierend elektrisch in Reihe geschaltet sind.

7. Verfolger (10) nach einem der vorangehenden Ansprüche,
umfassend einen Knotenpunkt (26) einer Mehrzahl von elektrischen Verbindungen (16).

8. Verfolger (10) nach einem der vorangehenden Ansprüche,
umfassend eine mit zumindest zwei der Verfolgerelemente (14, 15) elektrisch verbundene Steuerung (22), die eingerichtet ist, unabhängig den Betrieb der zumindest zwei Verfolgerelemente (14, 15) zu steuern.

9. Verfolger (10) nach einem der vorangehenden Ansprüche,
wobei das flächige Element (12) eine mäandernde Form aufweist.

10. Verfolger (10) nach einem der vorangehenden Ansprüche,
wobei zumindest ein Teil des flächige Elements (12) die Form eines Rahmens aufweist, der eine zentrale Öffnung (28) umschließt; und/oder
wobei die elektrischen Anschlüsse (16) auf das flächige Element (12) aufgedruckt sind.

11. Verfolger (10) nach einem der vorangehenden Ansprüche,
umfassend eine Mehrzahl von Schichten (18), wobei die elektrischen Verbindungen (16) in zumindest einer der Mehrzahl von Schichten (18) angeordnet sind;
wobei der Verfolger optional ein Durchgangsloch (20) umfasst, das eingerichtet ist, um zumindest zwei der Mehrzahl von Schichten (18) elektrisch zu verbinden.

12. Verfolger (10) nach einem der vorangehenden Ansprüche,
umfassend einen Klebstoff, der ausgebildet ist, um das flächige Element (12) an einer Oberfläche eines chirurgischen Objekts zu befestigen.

## Revendications

1. Suiveur (10) de navigation chirurgicale, le suiveur (10) comprenant
un timbre (12) étant au moins l'un parmi étirable et compressible dans au moins une direction;
une pluralité d'éléments suiveurs sélectionnés parmi une source de lumière (14) ou une bobine (15), les éléments suiveurs (14, 15) étant supportés par le timbre (12); et
une pluralité de connexions électriques (16) couplées électriquement aux éléments suiveurs (14, 15) et supportées par le timbre (12), les connexions électriques (16) ayant une configuration en méandres le long de la direction dans laquelle le timbre (12) est au moins l'un parmi étirable et compressible **caractérisé en ce que** le timbre (12) comprend de multiples doigts, dans lequel au moins deux des doigts supportent chacun au moins un élément suiveur et au moins une connexion électrique associée,

2. Suiveur (10) selon la revendication 1, dans lequel
le timbre (12) est déformable élastiquement.

3. Suiveur (10) selon la revendication 1 ou 2, dans lequel
la configuration en méandres comprend un motif périodique.

4. Suiveur (10) selon une quelconque revendication précédente, dans lequel
la configuration en méandres comprend au moins l'un parmi un motif en vague, un motif en vague rectangulaire et un motif en zigzag.

5. Suiveur (10) selon une quelconque revendication précédente, dans lequel les connexions électriques (16) ont une largeur entre 5 µm et 50 µm.

6. Suiveur (10) selon une quelconque revendication précédente, dans lequel au moins certains parmi les éléments suiveurs (14, 15) et les connexions électriques (16) sont couplés électriquement en série en alternance.

7. Suiveur (10) selon l'une quelconque des revendications précédentes comprenant une jonction (26) d'une pluralité de connexions électriques (16).

8. Suiveur (10) selon une quelconque revendication précédente,
comprenant un organe de commande (22° connecté électriquement à au moins deux des éléments suiveurs (14, 15) et configuré pour commander le fonctionnement des au moins deux des éléments suiveurs (14, 15) de manière indépendante.

9. Suiveur (10) selon une quelconque revendication précédente,
dans lequel le timbre (12) a une forme méandrique.

10. Suiveur (10) selon une quelconque revendication précédente,
dans lequel au moins une partie du timbre (12) a la forme d'un cadre renfermant une ouverture centrale (28); et/ou
dans lequel les connexions électriques (16) sont imprimées sur le timbre (12).

11. Suiveur (10) selon une quelconque revendication précédente,
comprenant une pluralité de couches (18), dans lequel les connexions électriques (16) sont disposées dans au moins l'une parmi la pluralité de couches (18);
dans lequel, éventuellement, le suiveur comprend un trou d'interconnexion (20) conçu pour connecter électriquement au moins deux parmi la pluralité de couches (18).

12. Suiveur (10) selon une quelconque revendication précédente,
comprenant un adhésif conçu pour fixer le timbre (12) à une surface d'un objet chirurgical.
